# EUROPÄISCHE PATENTSCHRIFT

(11) **EP 4 177 446 B1**
(45) Veröffentlichungstag und Bekanntmachung des Hinweises auf die Patenterteilung: **25.09.2024**
(21) Anmeldenummer: 22208617.5
(22) Anmeldetag: 23.10.2018
(51) Int. Cl.: F01K 3/18, F01K 23/06

(54) **VERFAHREN ZUM BETREIBEN EINER CHEMISCHEN ANLAGE**
METHOD FOR OPERATING A CHEMICAL INSTALLATION
PROCÉDÉ DE FONCTIONNEMENT D'UNE INSTALLATION CHIMIQUE

(43) Veröffentlichungstag der Anmeldung: 10.05.2023
(62) Teilanmeldung aus: 18202126.1
(73) Patentinhaber: Balthasar, Wolff, 40833 Ratingen (DE); Koss, Ulrich, 61348 Bad Homburg vor der Höhe (DE)
(72) Erfinder: Balthasar, Wolff, 40833 Ratingen (DE); Koss, Ulrich, 61348 Bad Homburg vor der Höhe (DE)
(74) Vertreter: Patentanwälte Bauer Vorberg Kayser

(56) Entgegenhaltungen:
- EP-A1- 2 395 066
- US-A1- 2010 170 247
- US-B1- 6 258 860

## Beschreibung

Die Erfindung betrifft ein Verfahren zum Betreiben einer chemischen Anlage sowie eine chemische Anlage.

Die Stromerzeugung durch Dampfturbinen ist aus dem Stand der Technik wohlbekannt. Eine solche Dampfturbine weist regelmäßig mehrere Druckturbinenstufen auf, welche auf einer gemeinsamen Welle angeordnet sind, welche wiederum einen Generator zur Stromerzeugung antreibt. Der zugeführte und unter hohem Druck stehende Dampf passiert nun sukzessive die einzelnen Druckturbinenstufen zum Antreiben der Welle, wobei durch das Durchlaufen der einzelnen Stufen ein Druckverlust des Dampfes eintritt.

Ebenfalls aus dem Stand der Technik ist es bekannt, den Dampf nicht nur vor der erstmaligen Zufuhr zu der Dampfturbine - und damit also zu der ersten Druckturbinenstufe -, sondern auch zwischen der ersten und der unmittelbar nachgelagerten Druckturbinenstufe zu überhitzen, um auf diese Weise den Wirkungsgrad der Dampfturbine zu verbessern. Eine solche Überhitzung ermöglicht es, in der letzten Druckturbinenstufe durch eine Absenkung des Drucks fast bis zum Vakuum eine Kondensation des Abdampfes zu erreichen. Solche Kondensationsturbinen erreichen einen sehr hohen Wirkungsgrad, wobei bis zu 40 % der Gesamtleistung auf diese letzte Druckturbinenstufe zurückgehen können.

Dampfturbinen werden aber nicht nur in Kraftwerken zur Stromerzeugung eingesetzt, wo regelmäßig eine einzige Turbine eine sehr große Leistung erzeugt, sondern werden in kleinerer Dimensionierung auch in Anlagen der chemischen Industrie eingesetzt, in denen regelmäßig Prozessdampf anfällt, welcher in einer Dampfturbine verwerten werden kann. In solchen chemischen Anlagen treiben die Dampfturbinen häufig mechanische Vorrichtungen direkt ohne Zwischenschritt der Stromerzeugung an.

Es gibt eine Vielzahl von chemischen Anlagen, insbesondere Syntheseanlagen beispielsweise zur Herstellung von Methanol oder Ammoniak, in welchen aus einem kohlenstoffhaltigen Energieträgerstrom wie etwa Erdgas ein wasserstoffhaltiger Stoff wie etwa Wasserstoff oder Synthesegas hergestellt wird. Aus solchem Synthesegas kann dann ggf. wiederum der letztendlich gewünschte chemische Stoff erzeugt werden. Auch in diesen chemischen Anlagen werden insbesondere kleinere Dampfturbinen eingesetzt, mit welchen dann Pumpen und Kompressoren der chemischen Anlage betrieben werden können. Aus der Vielzahl der erforderlichen Pumpen und Kompressoren ergibt sich die Notwendigkeit, eine ebenso große Anzahl an Dampfturbinen vorzusehen. Da diese Dampfturbinen durch die jeweils kleinere erforderliche Leistung in ihren Dimensionen hinter den Dampfturbinen für die Stromerzeugung in Kraftwerken zurückbleiben, weisen sie auch einen vergleichsweise geringeren Wirkungsgrad auf.

Eine bevorzugte Art der Herstellung des Synthesegases in chemischen Anlagen ist die katalytische partielle Oxidation, welche auch als autotherme Reformierung bezeichnet wird. Allerdings weist bei der katalytischen partiellen Oxidation durch die Zufuhr von Sauerstoff in den Prozess das austretende Synthesegas einen so hohen Partialdruck an Kohlenstoffmonoxid auf, dass eine Verwendung des Synthesegases für ein Überhitzen des Dampfes für die Zufuhr zur Dampfturbine nach heutigem Stand der Materialwissenschaften nicht praktikabel erscheint. Denn dieser hohe Partialdruck an Kohlenstoffmonoxid würde zu einer raschen Zerstörung der Überhitzungsvorrichtung durch Metal Dusting führen.

Diese fehlende Möglichkeit der Überhitzung durch das an sich ausreichend heiße Synthesegas führt dazu, dass alle Hitze für die Überhitzung des Dampfes durch eine mit dem Erdgas befeuerte Heizvorrichtung - auch als fired heater bezeichnet - bereitgestellt werden muss. Das für das Befeuern eingesetzte Erdgas kann dann nicht für die Synthese verwendet werden, was die Ausbeute der Anlage verringert.

Die US 2010/170247 A1 aus dem Stand der Technik, von welcher die vorliegende Erfindung ausgeht, beschreibt ein kombiniertes Dampfturbinensystem. Das Dampfturbinensystem umfasst einen Gasifizierungsabschnitt, einen Methanisierungsabschnitt und einen Dampfturbinenabschnitt. Der Gasifizierungsabschnitt enthält eine neue Konstruktion zur Wärmerückgewinnung und die zughörigen Steuervorrichtungen zum Erhalten eines gewünschten Verhältnisses zwischen Dampf und trockenem Gas von 1,1 bis 2,2. Der Dampfturbinenabschnitt umfasst eine Hochdruck-, Mitteldruck- und Niederdruckdampfturbine mit einem Eingang, der mit einem Ausgang der Überhitzer im Methanisierungsprozess gekoppelt ist.

Ausgehend von diesem Stand der Technik besteht die Aufgabe der Erfindung folglich darin, eine Dampfturbine bei einer chemischen Anlage und ein Verfahren zum Betrieb einer solchen chemischen Anlage derart weiterzuentwickeln, dass die Dampfturbine ihre Leistung mit einem geringeren Verbrauch an Erdgas oder einem anderen Energieträger für die Überhitzung des Dampfes bereitstellen kann.

Bezogen auf ein Verfahren zum Betreiben einer chemischen Anlage wird diese Aufgabe durch ein Verfahren zum Betreiben einer chemischen Anlage mit den Merkmalen von Anspruch 1 gelöst. Bezogen auf eine chemische Anlage wird diese Aufgabe durch eine chemische Anlage mit den Merkmalen von Anspruch 15 gelöst.

Der Erfindung liegt die Erkenntnis zugrunde, dass eine erneute Überhitzung des Dampfes zwischen den Druckturbinenstufen - welcher Vorgang der erneuten Überhitzung auch als reheating bezeichnet wird - bei einer Dampfturbine im Kontext einer chemischen Anlage nicht nur im Bereich der hohen Drücke, also zwischen den ersten beiden Druckturbinenstufen, sondern auch im Bereich niedrigerer Drücke zwischen den jeweils nachfolgenden Druckturbinenstufen durchgeführt werden kann. Bei einem solchen Vorgehen führt die Überhitzung zwar zu einem etwas niedrigeren Wirkungsgrad als bei einer Überhitzung bei höheren Dampfdrücken, doch kann dann interne Prozesshitze der chemischen Anlage für die Überhitzung verwendet werden, sodass zumindest für diese Überhitzung die befeuerte Heizvorrichtung nicht erforderlich ist.

Im Ergebnis eröffnet sich durch eine solche erneute Überhitzung die Möglichkeit, die Vielzahl an kleineren Dampfturbinen der chemischen Anlage auf wirtschaftliche Art und Weise vollständig durch eine einzelne, entsprechend größere Dampfturbine zur Stromerzeugung zu ersetzen, wobei dann die vormals rein mechanisch angetriebenen Pumpen und Kompressoren der chemischen Anlage elektrisch angetrieben werden können. Die durch die Zwischenwandlung in elektrische Energie anfallenden Verluste werden dann durch den verbesserten Wirkungsgrad der Dampfturbine mindestens wettgemacht.

Die Unteransprüche 2 bis 6 betreffen besonders vorteilhafte Ausgestaltungen der chemischen Anlage, gemeinsam mit welcher die Dampfturbine gemäß der Erfindung betrieben werden kann. Insbesondere betrifft dies vorteilhafte Möglichkeiten, Hitze zum Überhitzen des Dampfes aus einem Prozessschritt der chemischen Anlage abzugreifen.

Die Unteransprüche 7 bis 9 wiederum betreffen die Möglichkeit, ein mehrmaliges Überhitzen bei der Dampfturbine vorzusehen.

Schließlich betreffen die Unteransprüche 11 bis 14 verschiedene Möglichkeiten, wie von der Dampfturbine erzeugter Strom innerhalb der chemischen Anlage besonders vorteilhaft verwendet werden kann.

Bevorzugte Ausgestaltungen und Merkmale der erfindungsgemäßen chemischen Anlage entsprechen bevorzugten Ausgestaltungen und Merkmalen des erfindungsgemäßen Verfahrens und umgekehrt.

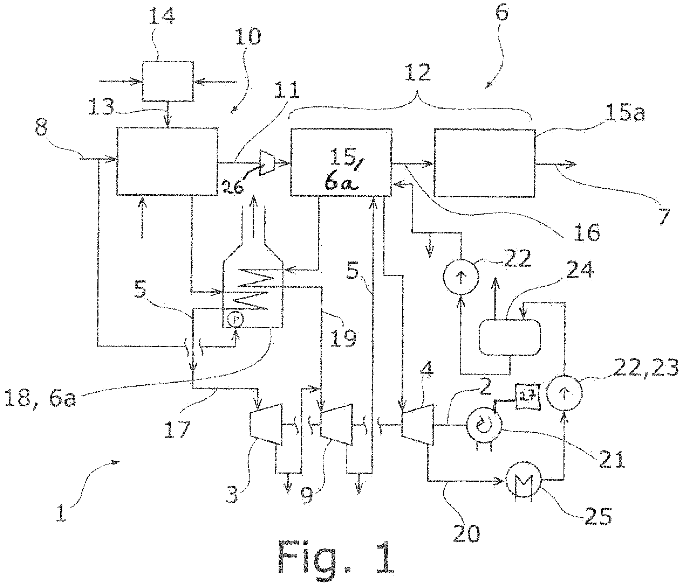

Weitere Einzelheiten, Merkmale, Ziele und Vorteile der vorliegenden Erfindung werden nachfolgend anhand einer lediglich ein Ausführungsbeispiel wiedergebenden Zeichnung erläutert. In der Zeichnung zeigt
- Fig. 1: eine schematische Darstellung eines Ausführungsbeispiels der vorschlagsgemäßen chemischen Anlage zur Ausführung des vorschlagsgemäßen Verfahrens.

Das vorschlagsgemäße Verfahren dient dem Betreiben einer vorschlagsgemäßen chemischen Anlage, welche hier in der Fig. 1 dargestellt ist. Gemäß dem vorschlagsgemäßen Verfahren weist die chemische Anlage eine Dampfturbine 1 mit einer Welle 2 und mit einer ersten Druckturbinenstufe 3 sowie einer zweiten Druckturbinenstufe 4 auf. Vorschlagsgemäß sind die erste Druckturbinenstufe 3 und die zweite Druckturbinenstufe 4 jeweils auf der Welle 2 angeordnet und dampfprozesstechnisch hintereinandergeschaltet. Die dampfprozesstechnische Hintereinanderschaltung der ersten Druckturbinenstufe 3 und der zweiten Druckturbinenstufe 4 bedeutet, dass die Dampfturbine 1 zum Antrieb der Welle 2 durchströmender Turbinendampf zunächst die erste Druckturbinenstufe 3 und danach erst die zweite Druckturbinenstufe 4 durchströmt. Dazwischen kann dieser Turbinendampf im Prinzip beliebig viele weitere Druckturbinenstufen der Dampfturbine 1 durchlaufen oder einem sonstigen Prozess zugeführt werden. Vorzugsweise weist die Dampfturbine 1 eine elektrische Maximalleistung von mindestens 30 MW und insbesondere eine elektrische Maximalleistung zwischen 50 MW und 200 MW auf.

Beim vorschlagsgemäßen Verfahren wird Dampf 5 zum Antreiben der Dampfturbine 1 aus einer Reaktoranlage 6 erhalten, welche Reaktoranlage 6 einen wasserstoffhaltigen Stoff 7 aus einem kohlenstoffhaltigen Energieträgerstrom 8 herstellt und wobei der Dampf 5 in einem Überhitzungsschritt vor Zufuhr zu der zweiten Druckturbinenstufe 4 erhitzt wird. Bevorzugt umfasst die chemische Anlage die Reaktoranlage 6. Vorschlagsgemäß wird der Dampf 5 auf eine Temperatur oberhalb der Sättigungstemperatur erhitzt. Es handelt sich dabei um die Sättigungstemperatur bei demjenigen Druck, welchen der Dampf 5 in dem Überhitzungsschritt aufweist. Ebenso kann die Erhitzung auf im Grunde beliebige Art und Weise erfolgen und durch eine im Grunde beliebige Energiequelle gespeist werden.

Der Dampf 5 ist bereits beim Erhalten aus der Reaktoranlage 6 und damit vor Zufuhr zu der Dampfturbine 1 - insbesondere vor Zufuhr zu der ersten Druckturbinenstufe 3 - erhitzt. Speziell ist bevorzugt, dass der Dampf 5 vor Zufuhr zu der Dampfturbine 1 - insbesondere vor Zufuhr zu der ersten Druckturbinenstufe 3 - auf eine Temperatur oberhalb der Sättigungstemperatur des Dampfes 5 erhitzt ist. Daher handelt es sich bei der Erhitzung vor Zufuhr zu der zweiten Druckturbinenstufe 4, zu welchem Zeitpunkt der Dampf 5 bereits der Dampfturbine 1 zugeführt wurde, um ein erneutes Erhitzen.

Gemäß dem vorschlagsgemäßen Verfahren weist die Dampfturbine 1 eine auf der Welle 2 angeordnete dritte Druckturbinenstufe 9 auf, welche dritte Druckturbinenstufe 9 dampfprozesstechnisch zwischen der ersten Druckturbinenstufe 3 und der zweiten Druckturbinenstufe 4 geschaltet ist. Das bedeutet, dass der aus der ersten Druckturbinenstufe 3 tretende Turbinendampf erst die dritte Druckturbinenstufe 9 durchströmt bevor er die zweite Druckturbinenstufe 4 durchströmt. Wiederum kann der Turbinendampf jeweils zwischen der ersten Druckturbinenstufe 3 und der dritten Druckturbinenstufe 9 und zwischen der dritten Druckturbinenstufe 9 und der zweiten Druckturbinenstufe 4 im Prinzip beliebig viele weitere Druckturbinenstufen durchlaufen oder einem sonstigen Prozess zugeführt werden.

Bei dem vorschlagsgemäßen Verfahren durchläuft der Dampf 5 den Überhitzungsschritt nach Austritt aus der dritten Druckturbinenstufe 9. Auf diese Weise findet also die Erhitzung des Dampfes 5 im Überhitzungsschritt vor Zufuhr zu der zweiten Druckturbinenstufe 4 statt.

Die vorschlagsgemäße chemische Anlage weist die Dampfturbine 1 auf, welche ihrerseits die Welle 2, die erste Druckturbinenstufe 3 und die zweite Druckturbinenstufe 4 aufweist, wobei die erste Druckturbinenstufe 3 und die zweite Druckturbinenstufe 4 jeweils auf der Welle 2 angeordnet und dampfprozesstechnisch hintereinandergeschaltet sind.

Die vorschlagsgemäße chemische Anlage weist weiter die Reaktoranlage 6 zur Herstellung des wasserstoffhaltigen Stoffs 7 aus dem kohlenstoffhaltigen Energieträgerstrom 8 auf, wobei Dampf 5 zum Antreiben der Dampfturbine 1 aus der Reaktoranlage 6 erhalten wird. Ebenso weist die vorschlagsgemäße chemische Anlage eine Erhitzungsanordnung 6a zum Erhitzen des Dampfes 5 vor Zufuhr zu der zweiten Druckturbinenstufe 4 auf. Grundsätzlich kann es sich bei dieser Erhitzungsanordnung 6a um eine beliebige Vorrichtung oder Gruppe von Vorrichtungen der chemischen Anlage handeln, welche wahlweise auch von der Reaktoranlage 6 umfasst sein können.

Bei der vorschlagsgemäßen chemischen Anlage weist die Dampfturbine 1 eine auf der Welle 2 angeordnete dritte Druckturbinenstufe auf 9, welche dampfprozesstechnisch zwischen der ersten Druckturbinenstufe 3 und der zweiten Druckturbinenstufe 4 geschaltet ist. Ferner erhitzt bei der vorschlagsgemäßen chemischen Anlage die Erhitzungsanordnung 6a den Dampf 5 nach Austritt aus der dritten Druckturbinenstufe 9. Es findet also eine Erhitzung zwischen der dritten Druckturbinenstufe 9 und der zweiten Druckturbinenstufe 4 stattfindet.

Vorzugsweise weist der Dampf 5 vor Zufuhr zu der Dampfturbine 1 - insbesondere vor Zufuhr zu der ersten Druckturbinenstufe 3 - eine Temperatur von mindestens 450° C auf. Damit ist der Dampf 5 überhitzt. Speziell kann der Dampf 5 vor Zufuhr zu der Dampfturbine 1 - vorzugsweise vor Zufuhr zu der ersten Druckturbinenstufe 3 - eine Temperatur zwischen 450° C und 600° C aufweisen. Entsprechend ist es bevorzugt, dass der Dampf 5 in dem Überhitzungsschritt mindestens auf die Temperatur des Dampfes 5 vor Zufuhr zu der Dampfturbine 1 - insbesondere vor Zufuhr zu der ersten Druckturbinenstufe 3 - erhitzt wird. Anders ausgedrückt wird der Dampf 5 in dem Überhitzungsschritt vorzugsweise auf eine Temperatur von mindestens 450° C und insbesondere auf eine Temperatur zwischen 450° C und 650° C erhitzt. Bezogen auf die chemische Anlage ist es daher ebenso bevorzugt, dass die Erhitzungsanordnung 6a den Dampf 5 auf eine Temperatur von mindestens 450° C und vorzugsweise auf eine Temperatur zwischen 450° C und 650° C erhitzt. Ebenso bevorzugt ist vorgesehen, dass der Dampf 5 bei Zufuhr zu der zweiten Druckturbinenstufe 4 einen Druck zwischen 1 bar und 20 bar aufweist. Insbesondere kann es sein, dass der Dampf 5 bei Zufuhr zu der zweiten Druckturbinenstufe 4 einen Druck zwischen 2 bar und 8 bar aufweist.

Da sich der Druck des Dampfes 5 beim sukzessiven Durchströmen der Druckturbinenstufen 3, 4, 9 verringert, kann die erste Druckturbinenstufe 3 auch als Hochdruck-Turbinenstufe, die der ersten Druckturbinenstufe 3 nachgeschaltete dritte Druckturbinenstufe 9 als Mitteldruck-Turbinenstufe und die zweite Druckturbinenstufe 4 als Niederdruck-Turbinenstufe bezeichnet werden. So kann beispielsweise der Dampf 5 vor Durchströmen der ersten Druckturbinenstufe 3 einen Druck zwischen 80 bar und 300 bar, vorzugsweise zwischen 100 bar und 200 bar, aufweisen. Ebenso ist es bevorzugt, dass der Dampf 5 nach Durchströmen der ersten Druckturbinenstufe 3 und vor Durchströmen der dritten Druckturbinenstufe 9 einen Druck zwischen 30 bar und 100 bar und schließlich nach Durchströmen der zweiten Druckturbinenstufe 4 einen Druck zwischen 0,01 und 0,1 bar aufweist. Der jeweils vor der ersten Druckturbinenstufe 3 und vor der zweiten Druckturbinenstufe 4 auf eine Temperatur von hier über 500° C überhitzte Dampf weist beim Austritt aus der zweiten Druckturbinenstufe 4 beispielhaft noch eine Temperatur zwischen 15° C und 40° C, vorzugsweise zwischen 20° C und 30° C, auf. Ebenso ist es bevorzugt, dass der Dampf beim Austritt aus der ersten Druckturbinenstufe 3 eine Temperatur von mindestens 280° C oder von im Wesentlichen 280° C aufweist.

Bei dem wasserstoffhaltigen Stoff 7 handelt es sich um Ammoniak. Vorschlagsgemäß stellt die Reaktoranlage 6 also Ammoniak her. Es ist bevorzugt, dass die Reaktoranlage 6 zusätzlich Methanol herstellt.

Regelmäßig wird die Reaktoranlage 6 in mehrere Abschnitte mit jeweils unterschiedlichen Funktionen unterteilt sein. Hier ist es bevorzugt, dass in einem Synthesegasabschnitt 10 der Reaktoranlage 6 Synthesegas 11, vorzugsweise aufweisend Wasserstoff und Kohlenstoffoxide, gewonnen wird. Weiter ist es bevorzugt, dass das gewonnene Synthesegas 11 einem dem Synthesegasabschnitt 10 nachgelagerten Umwandlungsabschnitt 12 der Reaktoranlage 6 zugeführt wird, in welchem Umwandlungsabschnitt 12 das gewonnene Synthesegas 11 in den wasserstoffhaltigen Stoff 7 umgewandelt wird. Das Synthesegas 11 weist vorzugsweise Wasserstoff und Kohlenstoffoxide auf oder besteht im Wesentlichen daraus. Daneben kann das Synthesegas 11 auch Stickstoff sowie kleinere Anteile von Edelgasen aufweisen oder insbesondere aus Wasserstoff, Kohlenstoffoxiden, Stickstoff und Edelgasen bestehen. Bevorzugt wird das Synthesegas 11 in dem Umwandlungsabschnitt 12 in Methanol und/oder Ammoniak umgewandelt. Für diese Umwandlung können auch weitere Ausgangsstoffe dem Umwandlungsabschnitt 12 zugeführt werden, beispielsweise Stickstoff für die Herstellung von Ammoniak. Dies kann insbesondere dann erfolgen, wenn das Synthesegas 11 Stickstoff nicht in ausreichender Menge enthält.

Bevorzugt ist, dass der kohlenstoffhaltige Energieträgerstrom 8 dem Synthesegasabschnitt 10 zum Gewinnen des Synthesegases 11 zugeführt wird, dass ein sauerstoffhaltiger Strom 13 dem Synthesegasabschnitt 10 zugeführt wird und dass das Synthesegas 11 in dem Synthesegasabschnitt 10 durch eine katalytische partielle Oxidation - welche auch als autotherme Reformierung bezeichnet werden kann - mittels des sauerstoffhaltigen Stroms 13 gewonnen wird. Wie in der Fig. 1 gezeigt kann es sein, dass der sauerstoffhaltige Strom 13 im Wesentlichen aus Sauerstoff besteht und aus einer Luftzerlegungsvorrichtung 14 der Reaktoranlage 6 gewonnen wird.

Bevorzugt ist weiter, dass der Dampf 5 durch Wärme aus einer Reaktion bei der Umwandlung des gewonnenen Synthesegases 11 in den wasserstoffhaltigen Stoff 7, vorzugsweise in Methanol und/oder Ammoniak, in dem Überhitzungsschritt erhitzt wird. So sind etwa die Reaktionen zum Bilden von Methanol aus Synthesegas 11 exotherm, ebenso wie die Reaktion zum Gewinnen von Ammoniak aus Wasserstoff und Stickstoff, wodurch also die Wärme zum Erhitzen des Dampfes gewonnen werden kann. In der Fig. 1 ist dargestellt, dass der Dampf 5 von der Dampfturbine 1 zur Erhitzung an den Umwandlungsabschnitt 12 - speziell an einen Reaktor 15 des Umwandlungsabschnitts 12 - und dann wieder zurück zur Dampfturbine 1 geführt wird. Entsprechend bildet hier der Reaktor 15 die Erhitzungsanordnung 6a. Dem Reaktor 15 nachgeschaltet ist gemäß der Darstellung der Fig. 1 eine Produktaufbereitung 15a des Umwandlungsabschnitts, welche aus einem aus dem Reaktor 15 tretenden Stoffstrom 16 den wasserstoffhaltigen Stoff 7 gewinnt. Die Produktaufbereitung 15a kann insbesondere zum Gewinnen des wasserstoffhaltigen Stroms 7 aus dem Stoffstrom 16 durch Reinigen des Stoffstroms 16 eingerichtet sein.

Vorzugsweise weist der Umwandlungsabschnitt 12 den Reaktor 15 mit einem Katalysator zur zumindest teilweisen Umwandlung des Synthesegases 11 in den wasserstoffhaltigen Stoff 7 auf. Ebenso kann der Umwandlungsabschnitt 12 einen - hier in der Fig. 1 nicht dargestellten - Wärmetauscher zum Abkühlen des Stoffstroms 16 aus dem Reaktor 15 aufweisen. Der Stoffstrom 16 weist einen Rohproduktstrom mit dem wasserstoffhaltigen Stoff 7 und ggf. mit nicht reagiertem Synthesegas auf. Weiter ist es bevorzugt, dass der Dampf 5 in dem Überhitzungsschritt durch Wärme aus dem Reaktor 15 oder dem Wärmetauscher erhitzt wird.

Regelmäßig ist der der ersten Druckturbinenstufe 3 zugeführte Dampf 5 bereits überhitzt und gesättigt. Daher wird vorzugsweise ein erster gesättigter und überhitzter Dampfstrom 17 der ersten Druckturbinenstufe 3 zum Antrieb der Dampfturbine 1 zugeführt. Grundsätzlich kann dieser erste Dampfstrom 17 in einem beliebigen Verhältnis zu dem Dampf 5 stehen. Insbesondere kann der erste Dampfstrom 17 separat zum Dampf 5 sein. Der Dampfstrom 17 kann aber auch den Dampf 5 umfassen oder aus ihm bestehen.

Für die Überhitzung des Dampfes 5 für die erste Druckturbinenstufe 3 ist regelmäßig eine höhere Temperatur erforderlich als für die nachgelagerte Überhitzung zwischen der dritten Druckturbinenstufe 9 und der zweiten Druckturbinenstufe 4. Daher sieht das hier in der Fig. 1 gezeigte Ausführungsbeispiel vor, dass die Reaktoranlage 6 eine befeuerte Heizvorrichtung 18 aufweist, welche den Dampf 5, welcher gesättigt aus der Reaktoranlage 6 erhalten wird, zum Erhalten des ersten Dampfstroms 17 überhitzt. Die befeuerte Heizvorrichtung 18 kann neben diesem Überhitzen des Dampfes 5 auch weitere Funktionen haben.

Bevorzugt ist weiter, dass die Heizvorrichtung 18 durch den kohlenstoffhaltigen Energieträgerstrom 8 gespeist wird. Ebenso ist es bevorzugt, dass der Dampf 5 gesättigt aus dem Synthesegasabschnitt 10 erhalten wird. Der Dampf 5 kann beispielsweise aus einer Entwässerung des Synthesegasabschnitts 10 erhalten werden.

Die Dampfturbine 1 kann einerseits so betrieben werden, dass alle ihre Druckturbinenstufen 3, 4, 9 im Wesentlichen nur durch denjenigen Dampf 5 betrieben werden, welcher bereits der ersten Druckturbinenstufe 3 zugeführt wird. Es ist aber auch möglich - wie in der Fig. 1 dargestellt - nachgelagert zur ersten Druckturbinenstufe 3 zusätzlichen Dampf der Druckturbine 1 zuzuführen. Entsprechend ist es bevorzugt, dass aus der Reaktoranlage 6, und zwar hier speziell aus dem Umwandlungsabschnitt 12, ein zweiter, insbesondere gesättigter, Dampfstrom 19 erhalten wird, welcher von der Heizvorrichtung 18 überhitzt wird und welcher der dritten Druckturbinenstufe 9 zum Antrieb der Dampfturbine 1 zugeführt wird. Wie in der Fig. 1 gezeigt ist es bevorzugt, dass aus der ersten Druckturbinenstufe 3 tretender Dampf 5 der dritten Druckturbinenstufe 9 zum Antrieb der Dampfturbine 1 zugeführt wird. Entsprechend kann es sein, dass der zweite Dampfstrom 19 mit dem ersten Dampfstrom 17 zusammengeführt wird, nachdem der erste Dampfstrom 17 aus der ersten Druckturbinenstufe 3 getreten ist. Regelmäßig treten im Umwandlungsabschnitt 12 Drücke auf, welche geringer als diejenigen im Syntheseabschnitt 10 sind. Daher kann der gegenüber dem ersten Dampfstrom 17 einen geringeren Druck aufweisende zweite Dampfstrom 19 aus dem Umwandlungsabschnitt 12 vorteilhafterweise mit dem ersten Dampfstrom 17 dann zusammengeführt werden, wenn der erste Dampfstrom 17 bereits etwas Druck durch Durchströmen der ersten Druckturbinenstufe 3 verloren hat.

Ebenso kann es sein, dass ein Prozessdampfstrom, bei welchem es sich vorzugsweise um einen Teilstrom des Dampfers 5 handelt, nach Austreten aus der ersten Druckturbinenstufe 3 oder aus der dritten Druckturbinenstufe 9 entnommen wird. Vorzugsweise wird der Prozessdampfstrom vor Zufuhr zu der dritten Druckturbinenstufe 9 oder vor Zufuhr zu der zweiten Druckturbinenstufe 4 entnommen. Ein solcher entnommener Prozessdampfstrom wird bevorzugt der Reaktoranlage 6 zugeführt. Insbesondere kann der entnommene Prozessdampfstrom der Reaktoranlage 6 und speziell dem Synthesegasabschnitt 10 oder dem Umwandlungsabschnitt 12 und dabei insbesondere der Produktaufbereitung 15a zugeführt werden. So kann die Produktaufbereitung 15a etwa Destillationskolonnen zur Produktaufbereitung umfassen. Diese benötigen regelmäßig größere Dampfmengen, welche entsprechend durch den entnommenen Prozessdampfstrom bereitgestellt werden können. Der entnommene Prozessdampfstrom kann insbesondere als Heizmedium und/oder als Reaktionsmedium in einem chemischen Prozess zugeführt werden.

Dann findet vorzugsweise eine Vermischung des Prozessdampfstroms mit einem Prozessstrom der Reaktoranlage 6 statt. Alternativ kann der Prozessdampfstrom als Heizmedium in einem Reboiler der chemischen Anlage verwendet werden. Vorzugsweise kondensiert der Prozessdampfstrom dabei und wird als Kondensat dem kondensierten Wasser aus dem - unten noch näher beschriebenen - Kondensator 25 zugeführt. Daraus folgt, dass der entnommene Prozessdampfstrom für eine Rückführung zur Dampfturbine 1 nicht mehr in Frage kommt.

Wie in der Fig. 1 dargestellt kann es sein, dass die Dampfturbine 1 eine Kondensationsturbine ist, sodass Kondensation im Abdampf insbesondere der zweiten Druckturbinenstufe 4 eintritt. Entsprechend ist es bevorzugt, dass die zweite Druckturbinenstufe 4 den ihr zugeführten Dampf 5 bis zu einem Nassdampf 20 entspannt. Dies erlaubt es, einen sehr hohen Wirkungsgrad mit der Dampfturbine 1 zu erreichen.

Ebenso gemäß der Darstellung in der Fig. 1 ist es bevorzugt, dass die chemische Anlage einen Generator 21 zur Erzeugung eines elektrischen Turbinenstroms umfasst, welcher Generator 21 von der Welle 2 angetrieben wird. Wird, wie vorliegend, eine Dampfturbine 1 und insbesondere eine Kondensationsturbine mit mehreren Druckturbinenstufen 3, 4, 9 betrieben, so kann mit ihr - und damit mit einer einzigen Dampfturbine 1 - ausreichend elektrische Leistung bereitgestellt werden, um alle oder zumindest alle wesentlichen elektrischen Verbraucher der Reaktoranlage 6 zu betreiben. Ggf. kann sogar durch den Generator 21 erzeugte überschüssige elektrische Leistung an andere Verbraucher jenseits der Reaktoranlage 6 bereitgestellt werden. Es ist dann nicht mehr erforderlich, eine Vielzahl von Dampfturbinen mit jeweils geringerer Leistung zu verwenden.

Grundsätzlich kann der Turbinenstrom für einen beliebigen Zweck eingesetzt werden. Bevorzugt ist jedoch, dass der Turbinenstrom die Luftzerlegungsvorrichtung 14 der Reaktoranlage 6 antreibt. Ebenso kann es sein, dass der Turbinenstrom eine Kompressoranordnung 26 und/oder eine Pumpenanordnung 22 der Reaktoranlage 6 antreibt.

Es kann beispielsweise auch vorteilhaft sein, dass die Luftzerlegungsvorrichtung 14 elektrisch angetrieben wird und sie zusätzlich oder ausschließlich mit Strom aus einem Stromnetz betrieben werden kann. Ebenso kann es sein, dass die Luftzerlegungsvorrichtung 14 zumindest zeitweilig Sauerstoff für den sauerstoffhaltigen Strom 13 und darüber hinaus einen Sauerstoffüberschuss produziert. Mit anderen Worten produziert die Luftzerlegungsvorrichtung 14 dann mehr Sauerstoff, als von der chemischen Anlage und insbesondere von dem Synthesegasabschnitt 10 benötigt wird. Vorzugsweise wird der Sauerstoffüberschuss dann insbesondere in flüssiger Form in einer geeigneten Speicheranlage zwischengespeichert.

Dies ermöglicht es, dass zu denjenigen Zeiten bzw. Tageszeiten gezielt zusätzlich Strom aus dem Stromnetz für die Luftzerlegungsvorrichtung 14 verbraucht werden kann, zu denen der Strompreis niedrig oder sogar negativ ist, während die Reaktoranlage 6 und somit auch die chemische Anlage weiterhin kontinuierlich betrieben werden können. In Zeiten höheren Strompreises kann dann der zwischengespeicherte Sauerstoffüberschuss dazu verwendet werden, die Sauerstoffzerlegungsleistung und damit den Stromverbrauch der Luftzerlegungsvorrichtung 14 abzusenken. Auf diese Weise fällt ein Stromüberschuss in dem Generator 21 an, der an das Stromnetz abgegeben werden kann. Eine derartig ausgestattete chemische Anlage kann mithin die Aufgabe eines Puffers, auch als peak shavers bezeichnet, übernehmen der dazu beiträgt, die Produktions- und Lastschwankungen eines Stromnetzes auszugleichen, wie sie durch das Einbinden der regenerativen Energiequellen Wind und Photovoltaik auftreten können. Auf die hier beschriebene Weise können vergleichsweise große Mengen an Energie in Form von Sauerstoff gespeichert werden. Diese Energie kann dann gezielt an das Stromnetz zurückgegeben werden, indem die Last der Luftzerlegungsvorrichtung 14 reduziert wird.

Hinsichtlich der Pumpenanordnung 22 kann es sein, dass die Pumpenanordnung 22 eine Heizkesselwasserpumpe 23 zum Bereitstellen von Wasser für einen Heizkessel 24 der Reaktoranlage 6 aufweist. Dieser Heizkessel 24 kann von dem Umwandlungsabschnitt 12 umfasst sein. Gemäß der Darstellung der Fig. 1 kann es sein, dass die Heizkesselwasserpumpe 23 mit Wasser aus dem Kondensator 25 der chemischen Anlage gespeist wird, welchem Kondensator 25 der Nassdampf 20 zugeführt wird.

Hinsichtlich der Kompressoranordnung 26 ist es bevorzugt, dass die Kompressoranordnung 26 einen Synthesegaskompressor zur Druckerhöhung in der Reaktoranlage 6 aufweist. Wie in der Fig. 1 dargestellt dient die Kompressoranordnung 26 und speziell der Synthesegaskompressor bevorzugt der Druckerhöhung des Synthesegases 11, und zwar insbesondere vor Zufuhr zu dem Reaktor 15.

Regelmäßig werden die Kompressoren einer Kompressoranordnung 26 und die Pumpen einer Pumpenanordnung 22 durch Elektromotoren angetrieben. Wenn diese durch den Strom aus dem elektrischen Stromversorgungsnetz gespeist werden, gestaltet sich die Einstellung ihrer Drehzahl schwierig. Denn die Drehzahl eines Elektromotors ist zunächst von der Frequenz des Stromes abhängig, welche bei dem Stromversorgungsnetz fest mit z. B. 50 Hz vorgegeben ist. Das Vorsehen eines mechanischen Schaltgetriebes zur Einstellung der jeweiligen Drehzahl ist teuer und erfordert eine sowohl komplexe als auch mühsam zu wartende Konstruktion.

Daher ist für einen effektiven Antrieb der Kompressoranordnung 26 und der Pumpenanordnung 22 bevorzugt vorgesehen, dass die chemische Anlage eine Frequenzumrichteranordnung 27 aufweist, welche insbesondere mit Leistungselektronik der Frequenzumrichteranordnung 27 den Turbinenstrom wandelt, und zwar vorzugsweise zum Antrieb der Kompressoranordnung 26 und/oder der Pumpenanordnung 22. Insbesondere kann die chemische Anlage eine Frequenzumrichteranordnung 27 mit einer einstellbaren Ausgangsfrequenz aufweisen. Dabei kann die Frequenzumrichteranordnung 27 auch eine Vielzahl von einzelnen Frequenzumrichtern aufweisen, so etwa je mindestens ein eigener Frequenzumrichter für die Kompressoranordnung 26, die Pumpenanordnung 22 und für die Luftzerlegungsvorrichtung 14 vorgesehen ist. Auf diese Weise werden mechanische Schaltgetriebe für diese Vorrichtungen entbehrlich. Es hat sich herausgestellt, dass durch Fortschritte bei Frequenzumrichtern trotz gegebener elektrischer Verluste und hoher Kosten im Ergebnis diese Lösung gegenüber mechanischen Schaltgetriebe wirtschaftlich vorteilhafter ist.

Das Vorsehen der Frequenzumrichteranordnung 27 erlaubt es ferner, beim Aufstarten der Reaktoranlage 6, wenn also noch nicht ausreichend Dampf 5 aus der Reaktoranlage 6 zum Betrieb der Dampfturbine 1 vorhanden ist, die Frequenzumrichteranordnung und damit auch die Kompressoranordnung 26, die Pumpenanordnung 22 und/oder die Luftzerlegungsvorrichtung 14 mit Strom aus dem Stromversorgungsnetz zu betreiben.

## Patentansprüche

1. Verfahren zum Betreiben einer chemischen Anlage, wobei die chemische Anlage eine Dampfturbine (1) mit einer Welle (2) und mit einer ersten Druckturbinenstufe (3) sowie einer zweiten Druckturbinenstufe (4), welche erste Druckturbinenstufe (3) und zweite Druckturbinenstufe (4) jeweils auf der Welle (2) angeordnet und dampfprozesstechnisch hintereinandergeschaltet sind, aufweist, wobei Dampf (5) zum Antreiben der Dampfturbine (1) aus einer Reaktoranlage (6) erhalten wird, welche Reaktoranlage (6) einen wasserstoffhaltigen Stoff (7) aus einem kohlenstoffhaltigen Energieträgerstrom (8) herstellt, wobei der Dampf (5) in einem Überhitzungsschritt vor Zufuhr zu der zweiten Druckturbinenstufe (4) erhitzt wird, wobei die Dampfturbine (1) eine auf der Welle (2) angeordnete dritte Druckturbinenstufe (9) aufweist, welche dampfprozesstechnisch zwischen der ersten Druckturbinenstufe (3) und der zweiten Druckturbinenstufe (4) geschaltet ist und wobei der Dampf (5) den Überhitzungsschritt nach Austritt aus der dritten Druckturbinenstufe (9) durchläuft, **dadurch gekennzeichnet, dass** der Dampf (5) auf eine Temperatur oberhalb der Sättigungstemperatur erhitzt wird und dass die Reaktoranlage (6) Ammoniak herstellt.

2. Verfahren nach Anspruch 1, wobei die Reaktoranlage (6) Methanol und Ammoniak herstellt.

3. Verfahren nach Anspruch 1 oder 2, wobei in einem Synthesegasabschnitt (10) der Reaktoranlage (6) Synthesegas (11), vorzugsweise aufweisend Wasserstoff und Kohlenstoffoxide, gewonnen wird, insbesondere wobei das gewonnene Synthesegas (11) einem dem Synthesegasabschnitt (10) nachgelagerten Umwandlungsabschnitt (12) der Reaktoranlage (6) zugeführt wird, in welchem Umwandlungsabschnitt (12) das gewonnene Synthesegas (11) in den wasserstoffhaltigen Stoff (7) umgewandelt wird.

4. Verfahren nach Anspruch 3, wobei der kohlenstoffhaltige Energieträgerstrom (8) dem Synthesegasabschnitt (10) zum Gewinnen des Synthesegases (11) zugeführt wird, wobei ein sauerstoffhaltiger Strom (13) dem Synthesegasabschnitt (10) zugeführt wird und wobei das Synthesegas (11) in dem Synthesegasabschnitt (10) durch eine katalytische partielle Oxidation mittels des sauerstoffhaltigen Stroms (13) gewonnen wird, vorzugsweise, wobei der sauerstoffhaltige Strom (13) im Wesentlichen aus Sauerstoff besteht und aus einer Luftzerlegungsvorrichtung (14) der Reaktoranlage (6) gewonnen wird.

5. Verfahren nach Anspruch 4, wobei der Dampf (5) durch Wärme aus einer Reaktion bei der Umwandlung des gewonnenen Synthesegases (11) in den wasserstoffhaltigen Stoff (7) in dem Überhitzungsschritt erhitzt wird.

6. Verfahren nach Anspruch 4 oder 5, wobei der Umwandlungsabschnitt (12) einen Reaktor (15) mit einem Katalysator zur zumindest teilweisen Umwandlung des Synthesegases (11) in den wasserstoffhaltigen Stoff (7) aufweist, insbesondere, wobei der Umwandlungsabschnitt (12) einen Wärmetauscher zum Abkühlen eines Stoffstroms (16) aus dem Reaktor (15) aufweist, vorzugsweise, wobei der Dampf (5) in dem Überhitzungsschritt durch Wärme aus dem Reaktor (15) oder dem Wärmetauscher erhitzt wird.

7. Verfahren nach einem der Ansprüche 1 bis 6, wobei ein erster gesättigter und überhitzter Dampfstrom (17) der ersten Druckturbinenstufe (3) zum Antrieb der Dampfturbine (1) zugeführt wird.

8. Verfahren nach Anspruch 7, wobei die Reaktoranlage (6) eine befeuerte Heizvorrichtung (18) aufweist, welche den Dampf (5), welcher gesättigt aus der Reaktoranlage (6) erhalten wird, zum Erhalten des ersten Dampfstroms (17) überhitzt, vorzugsweise, welche Heizvorrichtung (18) durch den kohlenstoffhaltigen Energieträgerstrom (8) gespeist wird, insbesondere, wobei der Dampf (5) gesättigt aus dem Synthesegasabschnitt (10) erhalten wird.

9. Verfahren nach Anspruch 8, wobei aus der Reaktoranlage (6), vorzugsweise aus dem Umwandlungsabschnitt (12), ein zweiter, insbesondere gesättigter, Dampfstrom (19) erhalten wird, welcher von der Heizvorrichtung (18) überhitzt wird und welcher der dritten Druckturbinenstufe (9) zum Antrieb der Dampfturbine (1) zugeführt wird, weiter vorzugsweise, wobei aus der ersten Druckturbinenstufe (3) tretender Dampf (5) der dritten Druckturbinenstufe (9) zum Antrieb der Dampfturbine (1) zugeführt wird.

10. Verfahren nach einem der Ansprüche 1 bis 9, wobei die Dampfturbine (1) eine Kondensationsturbine ist, sodass Kondensation im Abdampf insbesondere der zweiten Druckturbinenstufe (4) eintritt, vorzugsweise, wobei die zweite Druckturbinenstufe (4) den ihr zugeführten Dampf bis zu einem Nassdampf (20) entspannt.

11. Verfahren nach einem der Ansprüche 1 bis 10, wobei die chemische Anlage einen Generator (21) zur Erzeugung eines elektrischen Turbinenstroms umfasst, welcher Generator (21) von der Welle (2) angetrieben wird, vorzugsweise, wobei der Turbinenstrom eine Kompressoranordnung (26) und/oder eine Pumpenanordnung (22), vorzugsweise auch die Luftzerlegungsvorrichtung (14), der Reaktoranlage (6) antreibt.

12. Verfahren nach Anspruch 11, wobei die Pumpenanordnung (22) eine Heizkesselwasserpumpe (23) zum Bereitstellen von Wasser für einen Heizkessel (24) der Reaktoranlage (6), vorzugsweise des Umwandlungsabschnitts (12), aufweist, insbesondere wobei die Heizkesselwasserpumpe (23) mit Wasser aus einem Kondensator (25) gespeist wird, welchem Kondensator (25) der Nassdampf (20) zugeführt wird.

13. Verfahren nach Anspruch 11 oder 12, wobei die Kompressoranordnung (26) einen Synthesegaskompressor zur Druckerhöhung in der Reaktoranlage (6), vorzugsweise zur Druckerhöhung des Synthesegases (11), insbesondere vor Zufuhr zu dem Reaktor (15), aufweist.

14. Verfahren nach einem der Ansprüche 11 bis 13, wobei die chemische Anlage eine Frequenzumrichteranordnung (27) aufweist, welche den Turbinenstrom, vorzugsweise zum Antrieb der Kompressoranordnung (26) und/oder der Pumpenanordnung (22), wandelt.

15. Chemische Anlage mit einer Dampfturbine (1), welche eine Welle (2), eine erste Druckturbinenstufe (3) und eine zweite Druckturbinenstufe (4) aufweist, wobei die erste Druckturbinenstufe (3) und die zweite Druckturbinenstufe (4) jeweils auf der Welle (2) angeordnet und dampfprozesstechnisch hintereinandergeschaltet sind, mit einer Reaktoranlage (6) zur Herstellung eines wasserstoffhaltigen Stoffs (7) aus einem kohlenstoffhaltigen Energieträgerstrom (8), wobei Dampf (5) zum Antreiben der Dampfturbine (1) aus der Reaktoranlage (6) erhalten wird und mit einer Erhitzungsanordnung (6a) zum Erhitzen des Dampfes (5) vor Zufuhr zu der zweiten Druckturbinenstufe (4), wobei die Dampfturbine (1) eine auf der Welle (2) angeordnete dritte Druckturbinenstufe (9) aufweist, welche dampfprozesstechnisch zwischen der ersten Druckturbinenstufe (3) und der zweiten Druckturbinenstufe (4) geschaltet ist, **dadurch gekennzeichnet, dass** die Erhitzungsanordnung (6a) den Dampf nach Austritt aus der dritten Druckturbinenstufe (9) auf eine Temperatur oberhalb der Sättigungstemperatur erhitzt und dass die Reaktoranlage (6) Ammoniak herstellt.

## Claims

1. A method for operating a chemical plant, wherein the chemical plant has a steam turbine (1) with a shaft (2) and with a first pressure turbine stage (3) as well as a second pressure turbine stage (4), the first pressure turbine stage (3) and second pressure turbine stage (4) respectively being disposed on the shaft (2) and respectively connected in series for the purposes of the steam process, wherein steam (5) for driving the steam turbine (1) is obtained from a reactor plant (6), the reactor plant (6) producing a hydrogen-containing material (7) from a carbon-containing energy carrier stream (8), wherein the steam (5) is heated in a superheating step before being supplied to the second pressure turbine stage (4), wherein the steam turbine (1) has a third pressure turbine stage (9) disposed on the shaft (2) which is connected for the purposes of the steam process between the first pressure turbine stage (3) and the second pressure turbine stage (4) and wherein the steam (5) passes through the superheating step after it exits the third pressure turbine stage (9), **characterized in that** the steam is heated to a temperature above the saturation temperature and **in that** the reactor plant (6) produces ammonia.

2. The method as claimed in claim 1, wherein the reactor plant (6) produces methanol and ammonia.

3. The method as claimed in claim 1 or claim 2, wherein synthesis gas (11), preferably comprising hydrogen and oxides of carbon, is obtained in a synthesis gas section (10) of the reactor plant (6), in particular wherein the synthesis gas (11) obtained is supplied to a conversion section (12) of the reactor plant (6) located downstream of the synthesis gas section (10), in which conversion section (12) the synthesis gas (11) obtained is converted into the hydrogen-containing material (7).

4. The method as claimed in claim 3, wherein the carbon-containing energy carrier stream (8) is supplied to the synthesis gas section (10) in order to obtain the synthesis gas (11), wherein an oxygen-containing stream (13) is supplied to the synthesis gas section (10) and wherein the synthesis gas (11) is obtained in the synthesis gas section (10) by means of a partial catalytic oxidation using the oxygen-containing stream (13), preferably wherein the oxygen-containing stream (13) substantially consists of oxygen and is obtained from an air separation device (14) of the reactor plant (6) .

5. The method as claimed in claim 4, wherein the steam (5) is heated in the superheating step by heat from a reaction during the conversion of the synthesis gas (11) obtained into the hydrogen-containing material (7) .

6. The method as claimed in claim 4 or claim 5, wherein the conversion section (12) has a reactor (15) with a catalyst for the at least partial conversion of the synthesis gas (11) into the hydrogen-containing material (7), in particular wherein the conversion section (12) has a heat exchanger for cooling a stream of material (16) from the reactor (15), preferably wherein the steam (5) is heated in the superheating step by heat from the reactor (15) or from the heat exchanger.

7. The method as claimed in one of claims 1 to 6, wherein a first saturated and superheated stream of steam (17) is supplied to the first pressure turbine stage (3) in order to drive the steam turbine (1).

8. The method as claimed in claim 7, wherein the reactor plant (6) has a fired heating device (18) which superheats the steam (5) which is obtained in a saturated condition from the reactor plant (6) in order to obtain the first stream of steam (17), preferably, the heating device (18) is fed by means of the carbon-containing energy carrier stream (8), in particular wherein the steam (5) is obtained from the synthesis gas section (10) in a saturated condition.

9. The method as claimed in claim 8, wherein a second, in particular saturated stream of steam (19) is obtained, preferably from the conversion section (12), and is superheated by the heating device (18) and is supplied to the third pressure turbine stage (9) in order to drive the steam turbine (1), more preferably wherein steam (5) exiting the first pressure turbine stage (3) is supplied to the third pressure turbine stage (9) in order to drive the steam turbine (1).

10. The method as claimed in one of claims 1 to 9, wherein the steam turbine (1) is a condensing turbine, so that condensation in the exhaust steam in particular enters the second pressure turbine stage (4), preferably wherein the second pressure turbine stage (4) decompresses the steam supplied to it down to wet steam (20) .

11. The method as claimed in one of claims 1 to 10, wherein the chemical plant comprises a generator (21) for the production of an electrical turbine current, the generator (21) being driven by the shaft (2), preferably wherein the turbine current drives a compressor assembly (26) and/or a pump assembly (22), preferably also the air separation device (14), of the reactor plant (6).

12. The method as claimed in claim 11, wherein the pump assembly (22) has a boiler water pump (23) for providing water for a boiler (24) of the reactor plant (6), preferably of the conversion section (12), in particular wherein the boiler water pump (23) is fed with water from a condenser (25) to which condenser (25) the wet steam (20) is supplied.

13. The method as claimed in claim 11 or claim 12, wherein the compressor assembly (26) has a synthesis gas compressor for raising the pressure in the reactor plant (6), preferably for raising the pressure of the synthesis gas (11), in particular prior to supply to the reactor (15).

14. The method as claimed in one of claims 11 to 13, wherein the chemical plant has a frequency converter assembly (27) which converts the turbine current, preferably in order to drive the compressor assembly (26) and/or the pump assembly (22).

15. A chemical plant with a steam turbine (1) which has a shaft (2), a first pressure turbine stage (3) and a second pressure turbine stage (4), wherein the first pressure turbine stage (3) and the second pressure turbine stage (4) are respectively disposed on the shaft (2) and in series for the purposes of the steam process, with a reactor plant (6) for the production of a hydrogen-containing material (7) from a carbon-containing energy carrier stream (8), wherein steam (5) in order to drive the steam turbine (1) is obtained from the reactor plant (6) and with a heating assembly (6a) for heating the steam (5) prior to supply to the second pressure turbine stage (4), wherein the steam turbine (1) has a third pressure turbine stage (9) disposed on the shaft (2), which is connected in series for the purposes of the steam process between the first pressure turbine stage (3) and the second pressure turbine stage (4), **characterized in that** the heating assembly (6a) heats the steam to a temperature above the saturation temperature after it exits the third pressure turbine stage (9) and **in that** the reactor plant (6) produces ammonia.

## Revendications

1. Procédé destinée à faire fonctionner une installation chimique, ladite installation chimique comportant une turbine à vapeur (1) pourvue d'un arbre (2) et d'un premier étage d'impulsion (3) ainsi que d'un deuxième étage d'impulsion (4), le premier étage d'impulsion (3) et le deuxième étage d'impulsion (4) étant disposé chacun sur l'arbre (2) et montés l'un derrière l'autre dans le processus à vapeur, la vapeur (5) qui entraîne la turbine à vapeur (1) étant obtenue à partir d'un système de réacteur (6), le système de réacteur (6) permettant d'obtenir un flux contenant de l'hydrogène (7) à partir d'un flux vecteur d'énergie contenant du carbone (8), la vapeur (5) étant chauffée, dans une étape de surchauffe, avant d'être introduite dans le deuxième étage d'impulsion (4), la turbine à vapeur (1) comportant un troisième étage d'impulsion (9) qui est disposé sur l'arbre (2) et monté, dans le processus à vapeur, entre le premier étage d'impulsion (3) et le deuxième étage d'impulsion (4), et la vapeur (5) étant soumis à ladite étape de surchauffe après avoir quitté le troisième étage d'impulsion (9), **caractérisé en ce que** la vapeur (5) est chauffée à une température supérieure à la température de saturation et que le système de réacteur (6) produit de l'ammoniac.

2. Procédé selon la revendication 1, le système de réacteur (6) produisant du méthanol et de l'ammoniac.

3. Procédé selon les revendications 1 ou 2, une partie à gaz de synthèse (10) du système de réacteur (6) permettant d'obtenir du gaz de synthèse (11), lequel comporte préférentiellement de l'hydrogène et des oxydes de carbone, le gaz de synthèse (11) ainsi obtenu étant notamment introduit dans une partie de transformation (12) du système de réacteur (6) laquelle est située en aval de la partie à gaz de synthèse (10 et dans laquelle le gaz de synthèse (11) obtenu est transformé de manière à obtenir la matière contenant de l'hydrogène (7) .

4. Procédé selon la revendication 3, le flux vecteur d'énergie contenant du carbone (8) étant introduit dans la partie à gaz de synthèse (10) pour obtenir le gaz de synthèse (11) tout en introduisant dans la partie à gaz de synthèse (10) un flux contenant de l'oxygène (13), le gaz de synthèse (11) étant obtenu au sein de la partie à gaz de synthèse (10) en mettant en œuvre une oxydation partielle catalytique par le flux contenant de l'oxygène (13), le flux contenant de l'oxygène (13) étant de préférence sensiblement constitué d'oxygène et obtenu à partir d'un système de décomposition de l'air (14) faisant partie du système de réacteur (6).

5. Procédé selon la revendication 4, la vapeur (5) étant chauffée, dans ladite étape de surchauffe, par la chaleur issue d'une réaction se produisant lorsque le gaz de synthèse (11) obtenu est transformé en matière contenant de l'hydrogène (7).

6. Procédé selon les revendications 4 ou 5, la partie de transformation (12) comportant un réacteur (15) pourvu d'un catalyseur destiné à transformer le gaz de synthèse (11) au moins partiellement en matière contenant de l'hydrogène (7), la partie de transformation (12) comportant notamment un échangeur de chaleur destiné à refroidir un flux (16) en provenance du réacteur (15), la vapeur (5) étant préférentiellement chauffée, dans ladite étape de surchauffe, par la chaleur en provenance du réacteur (15) ou de l'échangeur de chaleur.

7. Procédé selon l'une des revendications 1 à 6, un premier flux de vapeur saturé et surchauffé (17) étant introduit dans le premier étage d'impulsion (3) afin d'entraîner la turbine à vapeur (1).

8. Procédé selon la revendication 7, le système de réacteur (6) comportant un dispositif de chauffe (18) à foyer qui surchauffe la vapeur (5) qui est obtenue sous une forme saturée à partir du système de réacteur (6), pour ainsi obtenir un premier flux de vapeur (17), le dispositif de chauffe (18) étant préférentiellement alimenté par le flux vecteur d'énergie contenant du carbone (8), la vapeur (5) étant notamment obtenue à partir de la partie à gaz de synthèse (10) sous une forme saturée.

9. Procédé selon la revendication 8, un deuxième flux de vapeur (19) étant obtenu, notamment sous une forme saturée, à partir du système de réacteur (6), de préférence à partir de la partie de transformation (12), pour ensuite être surchauffé par le dispositif de chauffe (18) et être introduit dans le troisième étage d'impulsion (9) et ainsi entraîner la turbine à vapeur (1), la vapeur (5) sortant du premier étage d'impulsion (3) étant, de préférence encore, introduite dans le troisième étage d'impulsion (9) pour ainsi entraîner la turbine à vapeur (1).

10. Procédé selon l'une des revendications 1 à 9, **caractérisé en ce que** la turbine à vapeur (1) est une troisième turbine à condensation, la vapeur subissant donc une condensation, plus particulièrement celle qui quitte le deuxième étage d'impulsion (4), le deuxième étage d'impulsion (4) provoquant préférentiellement une détente de la vapeur qu'on y introduit, jusqu'à l'obtention de vapeur humide (20).

11. Procédé selon l'une des revendications 1 à 10, l'installation chimique comprenant un générateur (21) destiné à produire un courant électrique de turbine, le générateur (21) étant entraîné par l'arbre (2), le courant électrique de turbine faisant fonctionner, de préférence, un ensemble de compression (26) et/ou un ensemble de pompage (22), et préférentiellement aussi le dispositif de décomposition de l'air (14) faisant partie du système de réacteur (6).

12. Procédé selon la revendication 11, l'ensemble de pompage (22) comportant une pompe à eau de chaudière (23) servant à fournir de l'eau à une chaudière (24) faisant partie du système de réacteur (6), préférentiellement de la partie de transformation (12), la pompe à eau de chaudière (23) étant notamment alimentée par l'eau provenant d'un organe de condensation (25), la vapeur humide (20) étant introduite dans l'organe de condensation (25).

13. Procédé selon les revendications 11 ou 12, l'ensemble de compression (26) comportant un compresseur de gaz de synthèse destiné à augmenter la pression au sein du système de réacteur (6), préférentiellement destiné à augmenter la pression du gaz de synthèse (11), notamment avant que celui-ci ne soit introduit dans le réacteur (15) .

14. Procédé selon l'une des revendications 11 à 13, ladite installation chimique comportant un ensemble de variation de vitesse (27) qui fait varier le courant électrique de turbine, de préférence pour entraîner l'ensemble de compression (26) et/ou l'ensemble de pompage (22).

15. Installation chimique comportant une turbine à vapeur (1) qui est pourvue d'un arbre (2), d'un premier étage d'impulsion (3) et d'un deuxième étage d'impulsion (4), le premier étage d'impulsion (3) et le deuxième étage d'impulsion (4) étant disposés chacun sur l'arbre (2) et montés l'un derrière l'autre dans le processus à vapeur, un système de réacteur (6) qui permet d'obtenir un flux contenant de l'hydrogène (7) à partir d'un flux vecteur d'énergie contenant du carbone (8), de la vapeur (5) étant obtenue à partir du système de réacteur (6) et servant à entraîner la turbine à vapeur (1), et un ensemble de chauffage (6a) qui est destiné chauffer la vapeur (5) avant que celle-ci ne soit introduite dans le deuxième étage d'impulsion (4), la turbine à vapeur (1) comportant un troisième étage d'impulsion (9) qui est disposé sur l'arbre (2) et monté, dans le processus à vapeur, entre le premier étage d'impulsion (3) et le deuxième étage d'impulsion (4), **caractérisé en ce que** l'ensemble de chauffage (6a) vient chauffer la vapeur (5), après que celle-ci a quitté le troisième étage d'impulsion (9), à une température supérieure à la température de saturation et que le système de réacteur (6) produit du l'ammoniac.
